# EUROPÄISCHE PATENTSCHRIFT

(11) **EP 3 335 742 B1**
(45) Veröffentlichungstag und Bekanntmachung des Hinweises auf die Patenterteilung: **28.08.2019**
(21) Anmeldenummer: 17206879.3
(22) Anmeldetag: 13.12.2017
(51) Int. Cl.: A61M 1/16, A61L 2/18

(54) **VERFAHREN ZUR NACH- UND VORBEREITUNG VON THERAPIEABLÄUFEN AN EINER VORRICHTUNG ZUR EXTRAKORPORALEN BLUTBEHANDLUNG MIT KOMBINIERTER DESINFEKTION UND ENTKALKUNG MIT SAUREM KONZENTRAT**
METHOD FOR POST-PROCESSING AND PREPARATION OF THERAPY PROCESSES ON A DEVICE FOR EXTRACORPOREAL BLOOD TREATMENT WITH COMBINED DISINFECTING AND DESCALING USING ACIDIC CONCENTRATE
PROCÉDÉ DE POST-TRAITEMENT ET DE PRÉPARATION DE PROCESSUS THÉRAPEUTIQUES SUR UN DISPOSITIF DESTINÉ AU TRAITEMENT DU SANG EXTRACORPOREL AVEC DÉSINFECTION ET DÉCALCIFICATION COMBINÉS AU MOYEN D'UN CONCENTRÉ ACIDE

(30) Priorität: 16.12.2016 DE 102016124626
(43) Veröffentlichungstag der Anmeldung: 20.06.2018
(73) Patentinhaber: B. Braun Avitum AG, 34212 Melsungen (DE)
(72) Erfinder: Ritter, Kai-Uwe, 34212 Melsungen (DE)
(74) Vertreter: Winter, Brandl, Fürniss, Hübner, Röss, Kaiser, Polte - Partnerschaft mbB

(56) Entgegenhaltungen:
- EP-A1- 1 275 408
- DE-A1- 19 749 875
- DE-U1-202011 105 738
- US-A- 5 078 967
- US-A1- 2014 217 020

## Beschreibung

Die Erfindung betrifft ein Verfahren zum effizienten Durchführen von Desinfektions-, Entkalkungs- und Reinigungsvorgängen an einer Vorrichtung zur extrakorporalen Blutbehandlung, beispielsweise einer Dialysemaschine, zwischen zwei Therapieabläufen.

### Hintergrund der Erfindung

In Dialysezentren werden Vorrichtungen zur extrakorporalen Blutbehandlung für mehrere Patienten hintereinander benutzt, sodass zwischen zwei Therapieabläufen ein Desinfektionsprozess in der Vorrichtung zur extrakorporalen Blutbehandlung durchlaufen werden muss, um eine Verschleppung von Keimen zwischen zwei Patienten zu verhindern.

### Stand der Technik

Eine gängige Möglichkeit, die derzeit genutzt wird, um eine Vorrichtung zur extrakorporalen Blutbehandlung einsatzbereit für den nächsten Patienten zu machen, besteht in einer Heißdesinfektion des Dialysierflüssigkeitskreislaufs. Dabei wird die Vorrichtung bzw. deren Dialysierflüssigkeitskreislauf aufgeheizt, bis eine Temperatur von ca. 85°C erreicht ist, anschließend folgt eine Desinfektionsphase, bei der verschiedene Flusswege mit einem Desinfektionsmittel gespült werden, abschließend wird die Vorrichtung ausgespült und abgekühlt. Ein solcher Prozess hat eine Gesamtdauer von bis zu 40min. Während des Desinfektionsprozesses kann die Vorrichtung nicht genutzt werden, sodass die Dauer des Desinfektionsprozesses einen bestimmenden Faktor für die notwendige Zeitspanne zwischen zwei Therapieabläufen darstellt.

Ein gewöhnlicher Dialyseprozess findet unter Verwendung von Bicarbonat und saurem Konzentrat statt. Da der Einsatz von Bicarbonat zur Verkalkung der Maschinen beiträgt, müssen diese regelmäßig entkalkt werden. Ein übliches Mittel zur Entkalkung ist der Zusatz von Zitronensäure in das Desinfektionsmittel, auch bekannt unter dem Begriff "Citrothermischer Desinfektion", was den Vorteil einer kombinierten Lösung für Desinfektion und Entkalkung in einem Schritt bietet.

Da der Einsatz von heißer Zitronensäure allerdings auch an Edelstahl Korrosion verursacht, erfordert ihr Einsatz die Verwendung sehr hochwertiger, teurer Stähle. Zitronensäure ist für Patienten mit Heimdialyse zudem nicht ungefährlich und schwierig zu handhaben, da Zitronensäure klebrig und ätzend ist. Üblicherweise bleiben Behälter mit Zitronensäure an der Vorrichtung zur extrakorporalen Blutbehandlung angeschlossen, was aufwendige zusätzliche Schutzmaßnahmen in der Vorrichtung erzwingt, um ein Ansaugen von Zitronensäure während des Therapieablaufs zu verhindern.

Eine andere bekannte Methode zur Entkalkung ist das Spülen mit saurem Konzentrat, auch "Descaling" genannt. Dazu wird ein Volumen von ca. 200ml saurem Konzentrat von der Vorrichtung zur extrakorporalen Blutbehandlung angesaugt und unverdünnt durch den Hydraulikkreislauf gepumpt. Ein Aufheizen des Dialysierflüssigkeitskreislaufs ist nicht notwendig, sodass der zeitliche Aufwand reduziert wird.

Das Erfordernis von mehr als einer Chemikalie für die Prozessschritte des Desinfizierens und Entkalkens ist allerdings mit zusätzlichen Kosten für Material und Entsorgung verbunden. Zusätzlich wird die Handhabung der Chemikalien durch den Behältertausch erschwert. Bei einer Dialysemaschine wird beispielsweise das Entkalkungsmittel über die Konzentratansaugstäbe, die zum Ansaugen der sauren Komponente des Dialysierflüssigkeitsgemischs eingesetzt werden, angesaugt, was einen automatisierten Ablauf unmöglich macht. Bei einigen Entkalkungsmitteln liegt die Leitfähigkeit außerdem im Bereich des für die Dialysierflüssigkeitsmischung benötigten sauren Konzentrats, sodass ein Fehler bei einem versehentlich falsch angeschlossenen Behälter nicht unbedingt bemerkt wird.

Die Entfernung von Substanzen aus dem Blut während eines Dialyseprozesses können Verschmutzungen auf der Dialysierflüssigkeitsabflussseite verursachen, weshalb das Hydrauliksystem auch regelmäßig gereinigt werden muss. Ein übliches Mittel zur Entfernung von Verschmutzungen sind beispielsweise Hypochloridlösungen oder Peressigsäure. Eine Reinigung ist jedoch nicht nach jedem Therapieablauf erforderlich. Reinigungspatronen, die Natriumcarbonat als Reinigungsmittel einsetzen, sind ein derzeit gebräuchliches Mittel zur Reinigung von Vorrichtungen zur extrakorporalen Blutbehandlung.

Der Einsatz von Reinigungsmitteln auf Chlorbasis beim Prozessschritt der Reinigung und Entfettung fordert aufgrund seiner Aggressivität ein häufiges Wechseln der verwendeten Dialysierflüssigkeitsfilter, beispielsweise halten übliche Filter maximal 10 Reinigungszyklen aus. Ähnlich wie bei den Entkalkungsmitteln liegt bei einigen Desinfektionsmitteln die Leitfähigkeit außerdem im Bereich des für die Dialysierflüssigkeitsmischung benötigten sauren Konzentrats, sodass auch hier ein Fehler bei einem versehentlich falsch angeschlossenen Behälter nicht unbedingt bemerkt wird.

Bei der Verwendung einer Reinigungspatrone wird für jeden Einsatz dieselbe Menge an Soda verwendet, unabhängig von der tatsächlichen Verunreinigung. Dies führt einerseits zu einer unnötigen Belastung der Abwässer, andererseits ist die Reinigungspatrone als Disposable mit einer erhöhten Müllentwicklung verbunden. Zudem wird dem Benutzer der Vorrichtung zur extrakorporalen Blutbehandlung die Handhabung durch eine zusätzliche Betriebskomponente erschwert.

Aus dem Stand der Technik bekannt ist beispielsweise die in der US Patentschrift US 5,978,976 offenbarte Hämodialysemaschine, deren Reinigung und Desinfizierung mit einer Mixtur aus einer basische Komponente, die ein Chlorit-Ion enthält, und einer sauren Komponente, die Milchsäure enthält, erfolgen soll.

Weiter ist in der Druckschrift DE 197 49 875 A ein Verfahren zur Reinigung einer Versorgungsanlage einer Dialysestation offenbart, bei der eine erste Leitung zum Führen von Dialysewasser (Permeat) und eine zweite Leitung zum Führen von Natriumbicarbonat vorgesehen ist. Vor der Heißreinigung wird die zweite Leitung ebenfalls mit Permeat gefüllt, um eine Zersetzung des Natriumbicarbonats zu vermeiden, und Zitronensäure als Reinigungszusatz zugegeben.

In der US 2014/0217020 A1 ist ein Dialysegerät und ein Verfahren zu dessen Reinigung und Desinfizierung offenbart, bei dem unter Einsatz von Zitronensäure gereinigt und schließlich ein Spülschritt mit physiologischer Lösung durchgeführt wird, um Verunreinigungen wie partikuläre Rückstände auszuspülen.

DE 20 2011 105 738 U1 offenbart ein Verfahren, bei dem ein Dialysegerät durch Diamant-Elektrolyse und Einsatz von Natriumhypochlorit oder Wasserstoffperoxid gereinigt und desinfiziert wird.

In EP 1 275 408 A1 ist ein Verfahren zur Bestimmung eines Verkalkungsgrads bei einer Dialysemaschine bekannt, bei dem der Vorgang des Entkalkens mittels eines sauren Mediums vorgenommen, welches auch der Desinfektion dient.

### Kurze Beschreibung der Erfindung

Es ist daher Aufgabe der vorliegenden Erfindung, die vorgenannten Probleme bekannter Desinfektions-, Entkalkungs- und Reinigungsprozesse beim Nach- bzw. Vorbereiten eines Therapieablaufs an einer Vorrichtung zur extrakorporalen Blutbehandlung durch Schaffung eines material-, zeit- und kosteneffizienten sowie umweltschonenden Therapieablauf-Komplett-Vorbereitungsprozesses zu überwinden.

Diese Aufgabe wird erfindungsgemäß durch die Merkmalskombination des Anspruchs 1 gelöst. Vorteilhafte Weiterbildungen der Erfindung sind Gegenstände der beigefügten Unteransprüche.

Ein Grundgedanke der Erfindung besteht somit darin, auf den Einsatz aggressiver Substanzen und betreuungsintensiver Prozessschritte zu verzichten ohne dabei die Sicherheit des vorzubereitenden Therapieablaufs durch Verunreinigungs- und/oder Chemikalienrückstände einzuschränken. Dies wird durch die Erfindung ermöglicht, indem Chemikalien, die zur Herstellung des gebräuchlichen Dialysierflüssigkeitsgemischs bereits an der Vorrichtung vorhanden sind, in geeigneter Weise eingesetzt werden und somit eine effiziente Prozessführung an einer Vorrichtung zur extrakorporalen Blutbehandlung erlauben. Anders ausgedrückt soll in einem Hydrauliksystem/Dialysierflüssigkeitskreislauf einer Vorrichtung zur extrakorporalen Blutbehandlung ein chemikalienarmer, umweit- und materialschonender Therapieablauf-Komplett-Vorbereitungsprozess gefahren werden, in dem die Gefährdung des Benutzers einer solchen Vorrichtung, beispielsweise Klinikpersonal oder Patienten in Heimdialyse, und die Materialabnutzung von Vorrichtung und Filter durch den alternativen Einsatz milder, ungefährlicherer Chemikalien umgangen wird. Unter Hydrauliksystem sind sämtliche Schlauch- und Rohrverbindungen zu verstehen, die für den Dialysierflüssigkeitskreislauf eingesetzt werden, also der Hydraulikkreislauf einschließlich Zulauf- und Ablaufleitungen für Dialysierflüssigkeitskomponenten und/oder weitere Chemikalien.

Konkret ist die Verwendung eines sauren Konzentrats, das auch zur Anmischung der Dialysierflüssigkeit verwendet wird, zur kombinierten Desinfektion und Entkalkung in einem ersten Prozessschritt vorgesehen, der bei Bedarf gefolgt wird von einem zweiten Prozessschritt zur Reinigung mit Bicarbonat. Durch den Prozessschritt Reinigung wird der erste Prozessschritt Desinfizieren und Entkalken somit zu einem vollständigen Therapieablauf-Nachbereitungsprozess ergänzt. Um die Vorrichtung in den Ausgangszustand für einen nächsten Therapieablauf zu setzen, wird das Hydrauliksystem anschließend mit Dialysierflüssigkeit oder hochreinem Wasser (Permeat) befüllt und der Vorbereitungsprozess für den folgenden Therapieablauf damit abgeschlossen.

Insgesamt werden erfindungsgemäße Vorteile dahingehen erzielt, dass durch die parallele Desinfektion und Entkalkung in einem einzigen Prozessschritt die benötigte Zeit zum Ausführen beider Einzelprozessschritte reduziert wird. Zusätzlich wird die Anzahl der benötigten Chemikalien reduziert. Die Handhabung der Maschine wird für den Benutzer deutlich vereinfacht, da er nur noch einen Chemikalienbehälter vorhalten muss und diesen während des Prozessschritts Desinfizieren/Entkalken nicht auszutauschen braucht. Zudem ist das saure Konzentrat, im Regelfall bereits an die Vorrichtung angeschlossen, wobei das saure Konzentrat einen Anteil Acetat und/oder Citrat (Zitronensäure) enthalten kann.

Die anschließende Reinigung mit Bicarbonat stellt insofern auch eine deutliche Verbesserung dar, als Bicarbonat, das zur Anmischung der Dialysierflüssigkeit verwendet wird, im Regelfall bereits an die Vorrichtung angeschlossen ist und der Benutzer auf einen zusätzlichen Chemikalienvorrat verzichten kann. Insgesamt wird so die Erzeugung von Plastikmüll wie Kanistern oder Kartuschen deutlich reduziert, auch wird der Entsorgungsaufwand durch die anfallenden Chemieabfälle verringert. Bevorzugt weist das saure Konzentrat einen Zitronensäureanteil auf. Zitronensäure weist gegenüber Essigsäure eine verbesserte Kalklöslichkeit auf. Daher sind saure Konzentrate mit einem Acetat- und einem Citratanteil zur Entkalkung besser geeignet als ein rein acetatsaures Konzentrat.

Die Aufgabe wird gelöst durch ein Verfahren zur Nach- und Vorbereitung eines Therapieablaufs an einer Vorrichtung zur extrakorporalen Blutbehandlung, in dem die Verfahrensschritte Desinfizieren, Entkalken, Reinigen, Spülen und Befüllen eines Hydrauliksystems ausführbar sind und der Verfahrensschritt Befüllen als letzter Verfahrensschritt vor Beginn eines nächsten Therapieablaufs mit einer Dialysierflüssigkeit oder mit hochreinem Wasser durchgeführt wird. Erfindungsgemäß erfolgen die Verfahrensschritte Desinfizieren und Entkalken des Hydrauliksystems in einem Kombinationsverfahrensschritt gleichzeitig durch Einsatz eines sauren Konzentrats und/oder der Verfahrensschritt Reinigen des Hydrauliksystems erfolgt durch Einsatz einer Bicarbonatlösung. Dabei weist der Verfahrensschritt Reinigen die Teilverfahrensschritte Ansaugen der Bicarbonatlösung, Erhitzen und Zirkulieren der Bicarbonatlösung, Zirkulieren der Bicarbonatlösung und Ausspülen der Bicarbonatlösung auf, wobei ein Erhitzen auf mindestens 50°C vorgesehen ist.

In anderen Worten wird die Aufgabe gelöst durch ein Verfahren an einer Vorrichtung zur extrakorporalen Blutbehandlung, das zwischen zwei Therapieabläufen zur Nach- und Vorbereitung eines Therapieablaufs angewandt wird, in dem das Hydrauliksystem desinfiziert, entkalkt, optional gereinigt, optional gespült und im letzten Schritt mit Dialysierflüssigkeit befüllt wird, dadurch gekennzeichnet, dass mit einem sauren Konzentrat gleichzeitig desinfiziert und entkalkt wird und mit einer Bicarbonatlösung gereinigt wird. Vorteilhafterweise dient der Einsatz des sauren Konzentrats zwei essenziellen Prozessschritten gleichermaßen, sodass die notwendige Zeit zum Desinfizieren und Entkalken des Hydrauliksystems deutlich verkürzt wird. Ebenso bedeutet die Zusammenlegung von Desinfizieren und Entkalken eine Chemikalienersparnis. Das saure Konzentrat ist gegenüber Edelstahl wenig aggressiv und materialschonend. Ein Behältertausch zwischen Desinfizieren und Entkalken muss nicht stattfinden, sodass der Vorgang leicht zu automatisieren ist und außerdem mit einer Vereinfachung der Hydraulik einhergeht. Acetat und Citrat sind umweltschonende Einsatzmittel, die sich mit dem anfallenden Kalk unter Kohlensäureentwicklung zu Calciumacetat bzw. Calciumcitrat verbindet und somit einfach ausgespült werden kann. Das Reinigungsmittel Bicarbonat ist im Gebrauch der Vorrichtung zur extrakorporalen Blutbehandlung bereits vorgesehen und erspart somit die Bereitstellung einer weiteren Chemikalie. Gelöstes Bicarbonat ist basisch und daher für die meisten Stähle und Kunststoffe sehr gut verträglich, sodass der Dialysierflüssigkeitsfilter geschont und länger verwendet werden kann.

Bevorzugt weist der Kombinationsverfahrensschritt Desinfizieren und Entkalken die Teilverfahrensschritte Ansaugen des sauren Konzentrats, Erhitzen und Zirkulieren des sauren Konzentrats und Ausspülen des sauren Konzentrats auf. In dem Teilverfahrensschritt Ansaugen des sauren Konzentrats wird eine tatsächlich benötigte Menge saures Konzentrat angesaugt.

Die tatsächlich benötigte Menge saures Konzentrat entspricht der Menge, die gebraucht wird, um einerseits Verkalkungen zu lösen und zusätzlich das Hydrauliksystem zu desinfizieren. Die benötigte Menge ist abhängig von der vorhergehenden Therapiezeit und der für die Therapie eingestellten Dialysierflüssigkeitszusammensetzung, da die Verkalkungsneigung mit dem Verhältnis von Bicarbonat und dessen Qualität zu saurem Konzentrat und dessen Acetatanteil und/oder Citratanteil korreliert. Die Ansaugmenge kann anhand des verwendeten Konzentrats, Mischverhältnis, Verhältnis Bicarbonat/Konzentrat und der vorgehenden Dialysedauer ermittelt werden. Die benötigte Menge kann entweder vom Anwender selbst anhand einer Tabelle ermittelt und in die Gerätesteuerung eingegeben werden oder aber automatisch von der Gerätesteuerung berechnet und übernommen werden. Somit wird unabhängig von den Bedingungen des vorangehenden Therapieablaufs ermöglicht, den Verbrauch des sauren Konzentrats so niedrig wie möglich zu halten und dennoch für jeden Verfahrenszyklus die erforderliche Menge bereitzustellen.

Außerdem bevorzugt wird in dem Teilverfahrensschritt Erhitzen und Zirkulieren des sauren Konzentrats auf mindestens 80°C, vorzugsweise mindestens 85°C oder wenn möglich noch höher, erhitzt. Die Desinfektion findet bei heißen Temperaturen statt. Durch das Erhitzen wird der pH-Wert des sauren Konzentrats weiter abgesenkt, da die Dissoziation der Ionen erhöht wird. Für das Abtöten der Keime ist ein möglichst niedriger pH-Wert des sauren Konzentrats erstrebenswert, im kalten Zustand liegt dieser zwischen 1,8 und 2,5, typischerweise zwischen 2,0 und 2,3, mindestens aber zwischen 1 und 3.

Weiter bevorzugt wird der Teilverfahrensschritt Zirkulieren des sauren Konzentrats für eine tatsächlich benötigte Zirkulationszeit durchgeführt.

Abhängig von der Dauer des vorangegangenen Therapieablaufs und der Verkalkungsneigung der zuletzt eingesetzten Dialysierflüssigkeitszusammensetzung, ist für den Teilverfahrensschritt Zirkulieren des sauren Konzentrats eine Mindestdauer erforderlich, um eine vollständige Desinfizierung und Entkalkung zu gewährleisten, hierbei spielt insbesondere das Verhältnis von Bicarbonat zu saurem Acetat bzw. Citrat eine Rolle. Die erforderliche Zeit kann von der Gerätesteuerung automatisch berechnet und übernommen werden oder vom Anwender anhand einer Tabelle ermittelt und in die Gerätesteuerung eingegeben werden. Die vorab Berechnung der erforderlichen Zeit ermöglicht, dass dieser Teilverfahrensschritt in einer optimalen Dauer gefahren wird, sodass die vollständige Desinfizierung und Entkalkung erreicht wird und gleichzeitig nicht mehr Zeit und Energie aufgewandt werden müssen, als erforderlich. Besonders bevorzugt wird der Teilverfahrensschritt Ausspülen des sauren Konzentrats bis zum Erreichen eines definierten Grenzwerts durchgeführt.

Der Anteil des sauren Konzentrats im Spülmedium, das üblicherweise hochreines Wasser ist, wird über die Leitfähigkeit der Spülmedium-Konzentrat-Mischung gemessen, sodass das Erreichen des Konzentrat-Grenzwerts im Spülmedium durch Erreichen des Leitfähigkeits-Grenzwerts festgestellt wird. Die Kopplung der Spüldauer an das Erreichen des Grenzwerts stellt sicher, dass nach jedem individuellen Therapieablauf, unabhängig von vorangegangener Therapiedauer und Dialysierflüssigkeitszusammensetzung, der Restverbleib des sauren Konzentrats unterhalb einer maximal zulässigen Grenze liegt und gleichzeitig nicht mehr Spülmedium, Zeit und Energie aufgewandt werden müssen, als für die vollständige Desinfizierung und Entkalkung erforderlich.

Bevorzugt wird in dem Teilverfahrensschritt Ansaugen der Bicarbonatlösung eine tatsächlich benötigte Menge Bicarbonatlösung angesaugt.

Der Verfahrensschritt Reinigen muss nicht zwingend in jedem Verfahren zur Vorbereitung des Hydrauliksystems auf einen Therapieablauf durchgeführt werden. Die tatsächlich benötigte Menge Bicarbonat entspricht der Menge, die gebraucht wird, um im Hydrauliksystem Verschmutzungen wie beispielsweise Fett, die während der Blutbehandlung entstanden sind, zu lösen. Die erforderliche Konzentration des Bicarbonats ist davon abhängig, welche Behandlungszeit seit der letzten Reinigung vergangen ist. Die Ansaugmenge kann von der Gerätesteuerung automatisch berechnet und übernommen werden oder aber vom Anwender in die Gerätesteuerung eingegeben werden. Nach Ermittlung der benötigten Menge Bicarbonat wird dieses in Form einer Bicarbonatlösung von einer Pumpe angesaugt, auf das erforderliche Mischverhältnis gebracht und dem Hydrauliksystem zugeführt. Somit wird unabhängig vom Verschmutzungsgrad ermöglicht, den Verbrauch des Bicarbonats so gering wie möglich zu halten und dennoch eine vollständige Reinigung zu gewährleisten.

Bei Temperaturen ab 50°C zersetzt sich Bicarbonat in Wasser, Kohlenstoffdioxid und Soda. Soda hat eine reinigende Wirkung und löst Fette. Bei höheren Temperaturen wird die Reinigungs- und Entfettungswirkung weiter verstärkt, sodass die Bicarbonatlösung bevorzugt auf 85°C, besonders bevorzugt so hoch wie möglich knapp unterhalb des Siedepunkts der Bicarbonatlösung, erhitzt wird.

Besonders bevorzugt wird der Teilverfahrensschritt Zirkulieren der Bicarbonatlösung für eine tatsächlich benötigte Zirkulationszeit durchgeführt.

Abhängig von der vorangegangenen Behandlungszeit, in der keine Reinigung stattgefunden hat, ist für den Teilverfahrensschritt Zirkulieren der Bicarbonatlösung eine Mindestdauer erforderlich, um eine vollständige Reinigung und Entfettung zu gewährleisten. Die erforderliche Zeit kann von der Gerätesteuerung automatisch berechnet und übernommen werden oder vom Anwender in die Gerätesteuerung eingegeben werden. Die vorab Berechnung der erforderlichen Zeit ermöglicht, dass dieser Teilverfahrensschritt in einer optimalen Dauer gefahren wird, sodass die vollständige Reinigung und Entfettung erreicht wird und gleichzeitig nicht mehr Zeit und Energie aufgewandt werden müssen, als erforderlich.

Weiter bevorzugt wird der Teilverfahrensschritt Ausspülen der Bicarbonatlösung bis zum Erreichen eines definierten Grenzwerts durchgeführt.

Der Anteil des Bicarbonats im Spülmedium, das üblicherweise hochreines Wasser ist, wird über die Leitfähigkeit der Spülmedium-Bicarbonat-Mischung gemessen, sodass das Erreichen des Bicarbonat-Grenzwerts im Spülmedium durch Erreichen des Leitfähigkeits-Grenzwerts festgestellt wird. Die Kopplung der Spüldauer an das Erreichen des Grenzwerts stellt sicher, dass nach einer beliebigen vorangegangenen Behandlungszeit, in der keine Reinigung stattgefunden hat, der Restverbleib des Bicarbonats unterhalb einer maximal zulässigen Grenze liegt und gleichzeitig nicht mehr Spülmedium, Zeit und Energie aufgewandt werden müssen, als für die vollständige Entfettung und Reinigung erforderlich.

Bevorzugt wird in einem Fall, in dem der nächste Therapieablauf nicht direkt im Anschluss an das Verfahren folgt, das Hydrauliksystem mit sauren Konzentrat befüllt, das saure Konzentrat verbleibt in dem Hydrauliksystem und das Hydrauliksystem wird direkt vor Beginn des nächsten Therapieablaufs gespült und mit Dialysierflüssigkeit befüllt.

Vorteilhafterweise kann das saure Konzentrat aufgrund seiner Materialverträglichkeit gegenüber Edelstahl und Kunststoff für längere Zeit im Hydrauliksystem verbleiben. Dies ist besonders bei längeren Ruhezeiten der Vorrichtung, z.B. über Nacht oder über das Wochenende, vorteilhaft, denn durch den niedrigen pH-Wert wird das Keimwachstum gegenüber einer Befüllung mit Wasser deutlich reduziert. Vor Beginn des nächsten Therapieablaufs wird das saure Konzentrat aus dem Hydrauliksystem mit hochreinem Wasser ausgespült, anschließend mit Dialysierflüssigkeit befüllt und somit in den Ausgangszustand für den nächsten Therapieablauf gesetzt.

Außerdem bevorzugt wird nach Überschreiten einer definierten Zeitspanne, in der kein weiterer Therapieablauf stattgefunden hat, vor Beginn des nächsten Therapieablaufs der Verfahrensschritt Desinfizieren und Entkalken erneut durchgeführt.

Dies hat den Vorteil, dass eventuelles Keimwachstum, das während einer längeren Ruhezeit stattgefunden hat, durch erneute Anwendung des Verfahrensschritts Desinfizierung und Entkalken entfernt wird und der nächste Therapieablauf ohne Verunreinigung durch Keime stattfinden kann. Das Erfordernis einer erneuten Durchführung des Verfahrensschritts Desinfizieren und Entkalken kann entweder automatisch von der Gerätesteuerung erkannt und dem Anwender angezeigt werden oder vom Anwender anderweitig überwacht werden.

### Kurzbeschreibunq der Zeichnungen

Die Erfindung wird nachstehend anhand bevorzugter Ausführungsbeispiele unter Bezugnahme auf die beigefügten Zeichnungen näher beschrieben.
Fig. 1a zeigt ein Ablaufschema für die Teilverfahrensschritte des Verfahrensschritts Desinfizieren und Entkalken
Fig. 1b zeigt ein Ablaufschema für die Teilverfahrensschritte des Verfahrensschritts Reinigen
Fig. 2 zeigt ein Diagramm zum Aufheizvorgang im Teilverfahrensschritts Erhitzen und Zirkulieren des Verfahrensschritts Reinigen
Fig. 3a zeigt eine erste Variante des Verfahrens zum Nach- und Vorbereiten eines Therapieablaufs an einer Vorrichtung zur extrakorporalen Blutbehandlung zwischen zwei Therapieabläufen
Fig. 3b zeigt eine zweite Variante des Verfahrens zum Nach- und Vorbereiten eines Therapieablaufs an einer Vorrichtung zur extrakorporalen Blutbehandlung zwischen zwei Therapieabläufen
Fig. 3c zeigt eine dritte Variante des Verfahrens zum Nach- und Vorbereiten eines Therapieablaufs an einer Vorrichtung zur extrakorporalen Blutbehandlung zwischen zwei Therapieabläufen
Fig. 3d zeigt eine vierte Variante des Verfahrens zum Nach- und Vorbereiten eines Therapieablaufs an einer Vorrichtung zur extrakorporalen Blutbehandlung zwischen zwei Therapieabläufen
Fig. 4a zeigt eine erste Variante eines Verfahrens zur Nach- und Vorbereitung eines Therapieablaufs an einer Vorrichtung zur extrakorporalen Blutbehandlung zwischen zwei Therapieabläufen für einen Fall, in dem zwischen zwei Therapieabläufen eine längere Ruhezeit geplant ist
Fig. 4b zeigt eine zweite Variante des Verfahrens zur Nach- und Vorbereitung eines Therapieablaufs an einer Vorrichtung zur extrakorporalen Blutbehandlung zwischen zwei Therapieabläufen für einen Fall, in dem zwischen zwei Therapieabläufen eine längere Ruhezeit geplant ist

### Detaillierte Beschreibung bevorzugter Ausführungsbeispiele

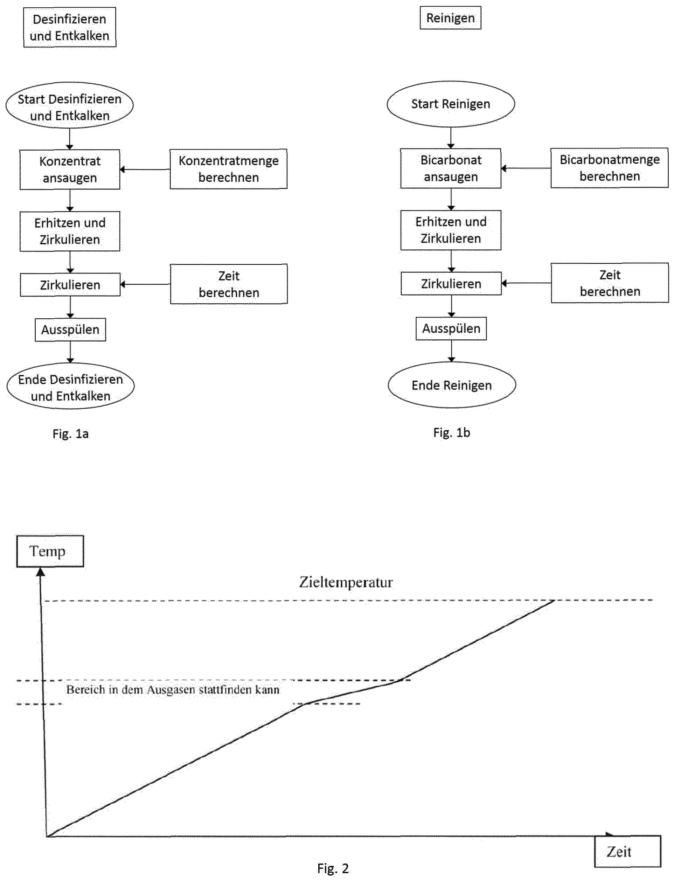

Fig. 1a zeigt in schematischer Darstellung den Ablauf der Teilverfahrensschritte des Verfahrensschritts Desinfizieren und Entkalken. Nachdem der Verfahrensschritt Desinfizieren und Entkalken gestartet wurde, wird zunächst die erforderliche Menge an sauren Konzentrat berechnet. Dies kann vom Anwender anhand eines Hilfsmittels vorgenommen werden, beispielsweise einer Tabelle, in der das verwendete saure Konzentrat, die Dialysierflüssigkeitszusammensetzung des vorangegangen Therapieablaufs, das Verhältnis von Bicarbonat zu saurem Konzentrat in der Dialysierflüssigkeit des vorangegangen Therapieablaufs und die vorgegangene Dialysezeit berücksichtigt werden. Der Anwender kann die ermittelte erforderliche Menge an saurem Konzentrat über die Gerätesteuerung eingeben. Alternativ kann die erforderliche Menge an saurem Konzentrat mithilfe der vorgenannten Parameter automatisch über die Gerätesteuerung berechnet werden.

Nachdem die erforderliche Menge an saurem Konzentrat ermittelt wurde, wird über eine Pumpe die erforderliche Menge an saurem Konzentrat aus dem Vorlagebehälter gesaugt, mit hochreinem Wasser, beispielsweise Revers Osmose (RO)-Wasser, auf das gewünschte Mischverhältnis gebracht und in das Hydrauliksystem der Vorrichtung zur extrakorporalen Blutbehandlung eingespeist. Da das saure Konzentrat auch für das Anmischen der Dialysierflüssigkeit verwendet wird, ist ein Austausch des Vorlagebehälters bzw. ein Anschließen des Behälters mit dem sauren Konzentrat bei ausreichender Restmenge nicht notwendig.

Die Konzentration des sauren Konzentrats aus dem Vorlagebehälter liegt bevorzugt zwischen 2mMol/Liter und 3mMol/Liter. Bevorzugter Weise liegt das saure Konzentrat aus dem Vorlagebehälter in einem Mischverhältnis mit hochreinem Wasser zwischen 1:12 und 1:8, besonders bevorzugt zwischen 1:11 und 1:9 und ganz besonders bevorzugt bei 1:10, sodass das eingespeiste saure Konzentrat eine Konzentration von ungefähr 0,2 bis 0,3 mMol/Liter hat.

Im Anschluss wird das eingespeiste saure Konzentrat im Hydraulikkreislauf zirkuliert und auf 80°C, bevorzugt 85°C und ganz besonders bevorzugt höher als 85°C erhitzt. Dabei löst das Acetat vorhandenen Kalk nach folgender Gleichung auf:

2 CH3COOH + CaCO3 → Ca(CH3COO)2 + H2CO3

Das gebildete Kalziumacetat wird anschließend ausgespült. Die gebildete Kohlensäure zerfällt nach folgender Gleichung in Kohlendioxid und Wasser:

H2CO3 → CO2 + H2O

Durch das Erhitzen erhöht sich die Dissoziation der Ionen, sodass der pH-Wert des sauren Konzentrats vorteilhafterweise weiter absinkt und das Abtöten von Keimen weiter begünstigt wird. Im kalten Zustand hat das saure Konzentrat einen pH-Wert zwischen 1 und 3, bevorzugt zwischen 1,8 und 2,5 und besonders bevorzugt zwischen 2,0 und 2,3.

Nachdem das saure Konzentrat seine Zieltemperatur erreicht hat, wird es für eine erforderliche Zeitspanne im Hydraulikkreislauf zirkulierend geführt. Die erforderliche Zeit wird aus den Parametern
- verwendetes saure Konzentrat,
- Dialysierflüssigkeitszusammensetzung des vorangegangen Therapieablaufs,
- Verhältnis von Bicarbonat zu saurem Konzentrat in der Dialysierflüssigkeit des vorangegangen Therapieablaufs und
- vorgegangene Dialysezeit
vorab berechnet. Der Anwender kann den berechneten Zeitwert in die Gerätesteuerung eingeben. Alternativ kann die Gerätesteuerung den erforderlichen Zeitwert anhand der vorgenannten Parameter automatisch berechnen und übernehmen. Die erforderliche Zeit ist die Dauer, über die das saure Konzentrat auf das Hydrauliksystem einwirken muss, um eine vollständige Desinfizierung und Entkalkung zu gewährleisten.

Abschließend wird das saure Konzentrat mit hochreinem Wasser ausgespült, bis ein festgelegter Grenzwert erreicht ist. Der Grenzwert wird über die Leitfähigkeit der zirkulierenden Flüssigkeit ermittelt. Nachdem der Grenzwert erreicht ist, ist der Verfahrensschritt Desinfizieren und Entkalken beendet.

Fig. 1b zeigt in schematischer Darstellung den Ablauf der Teilverfahrensschritte des Verfahrensschritts Reinigen. Nachdem der Verfahrensschritt Reinigen gestartet wurde, wird zunächst die erforderliche Menge an Bicarbonat berechnet. Dies kann vom Anwender anhand eines Hilfsmittels vorgenommen werden, beispielsweise einer Tabelle, in der die vorangegangene Therapiezeit, in der keine Reinigung vorgenommen wurde, berücksichtigt wird. Der Anwender kann die ermittelte erforderliche Menge an Bicarbonat über die Gerätesteuerung eingeben. Alternativ kann die erforderliche Menge an Bicarbonat mithilfe der Tabelle automatisch über die Gerätesteuerung berechnet werden.

Nachdem die erforderliche Menge an Bicarbonat ermittelt wurde, wird über eine Pumpe die erforderliche Menge an Bicarbonat aus dem Vorlagebehälter gesaugt, mit hochreinem Wasser, beispielsweise RO-Wasser, auf das gewünschte Mischverhältnis gebracht und in das Hydrauliksystem der Vorrichtung zur extrakorporalen Blutbehandlung eingespeist. Da Bicarbonat auch für das Anmischen der Dialysierflüssigkeit verwendet wird, ist ein Austausch des Vorlagebehälters bzw. ein Anschließen des Behälters mit dem Bicarbonat bei ausreichender Restmenge nicht notwendig.

Bicarbonat liegt im Vorlagebehälter entweder in gesättigter Lösung oder pulverförmig vor. Im Falle eines Pulvers wird das pulverförmige Bicarbonat vor Ansaugen durch die Pumpe mit hochreinem Wasser zu einer übersättigten Lösung angesetzt. Aus dem gesättigtem Überstand wird das Bicarbonat dann in gelöster Form eingespeist.

Im Anschluss wird das eingespeiste Bicarbonat im Hydraulikkreislauf zirkuliert und auf 50°C, bevorzugt 85°C und ganz besonders bevorzugt höher als 85°C bis kurz unter den Siedepunkte der Bicarbonatlösung erhitzt. Ab 50°C zersetzt sich das Bicarbonat in Wasser, Kohlenstoffdioxid und Soda. Die während des Aufheizvorgangs anfallende Menge Kohlenstoffdioxid kann über eine Vorrichtung zur Gasabscheidung abgeführt werden. Dabei wirkt es sich günstig aus, die Bicarbonatlösung vor Überschreiten des Temperaturbereichs, bei der das Ausgasen stattfindet, für eine bestimmte Dauer in diesem Temperaturbereich zu halten, um ein plötzliches schnelles Ausgasen zu verhindern. Dies wird in der Fig. 2 näher beschrieben. Das gebildete Soda ist die Waschsubstanz, mit der vorhandenes Fett und Verschmutzungen im Hydrauliksystem gelöst werden.

Nachdem das Bicarbonat seine Zieltemperatur erreicht hat, wird es für eine erforderliche Zeitspanne im Hydraulikkreislauf zirkulierend geführt. Dabei wird es mit möglichst hoher Flussrate zirkuliert. Die erforderliche Zeit wird aus der vorangegangenen Therapiezeit, in der keine Reinigung stattgefunden hat, vorab berechnet. Der Anwender kann den berechneten Zeitwert in die Gerätesteuerung eingeben. Alternativ kann die Gerätesteuerung den erforderlichen Zeitwert anhand des vorgenannten Parameters automatisch berechnen und übernehmen. Die erforderliche Zeit ist die Dauer, über die das Bicarbonat auf das Hydrauliksystem einwirken muss, um eine vollständige Entfettung und Reinigung zu gewährleisten.

Abschließend wird das Bicarbonat mit hochreinem Wasser ausgespült, bis ein festgelegter Grenzwert erreicht ist. Der Grenzwert wird über die Leitfähigkeit der zirkulierenden Flüssigkeit ermittelt. Nachdem der Grenzwert erreicht ist, ist der Verfahrensschritt Reinigen beendet.

In einer weiteren Ausführungsform ist es möglich, dass bereits beim Starten des gesamten Verfahrens die erforderlichen Zeiten berechnet werden, sodass die Dauer des gesamten Verfahrens bei Beginn angezeigt wird. Dies ermöglicht eine bessere Planung der nachfolgenden Therapieabläufe und trägt zu einem effizienten Betrieb des Dialysezentrums bei.

Fig. 2 zeigt ein Diagramm, in dem der Temperaturverlauf im Teilverfahrensschritt Erhitzen und Zirkulieren des Verfahrensschritts Reinigen in Abhängigkeit der Zeit aufgetragen ist. Mit den durchbrochenen Linien ist ein Temperaturbereich angedeutet, in dem Kohlenstoffdioxid aus der Bicarbonatlösung ausgast. Dieser Temperaturbereich beginnt bei etwa 50°C. Die Aufheizrate wird bei Erreichen des Temperaturbereichs reduziert und nach Überschreiten der oberen Grenze des Temperaturbereichs wieder gesteigert und bis zum Erreichen der Zieltemperatur beibehalten. Die Verringerung der Aufheizrate im Temperaturbereich des Ausgasens hat den Vorteil, dass die Bicarbonatlösung in diesem Temperaturbereich länger gehalten wird, als wenn die anfängliche Aufheizrate beibehalten würde. Eine längere Verweildauer der Bicarbonatlösung in diesem Temperaturbereich führt zu einer entschleunigten Ausgasung, mit der ein plötzliches schnelles Ausgasen verhindert wird.

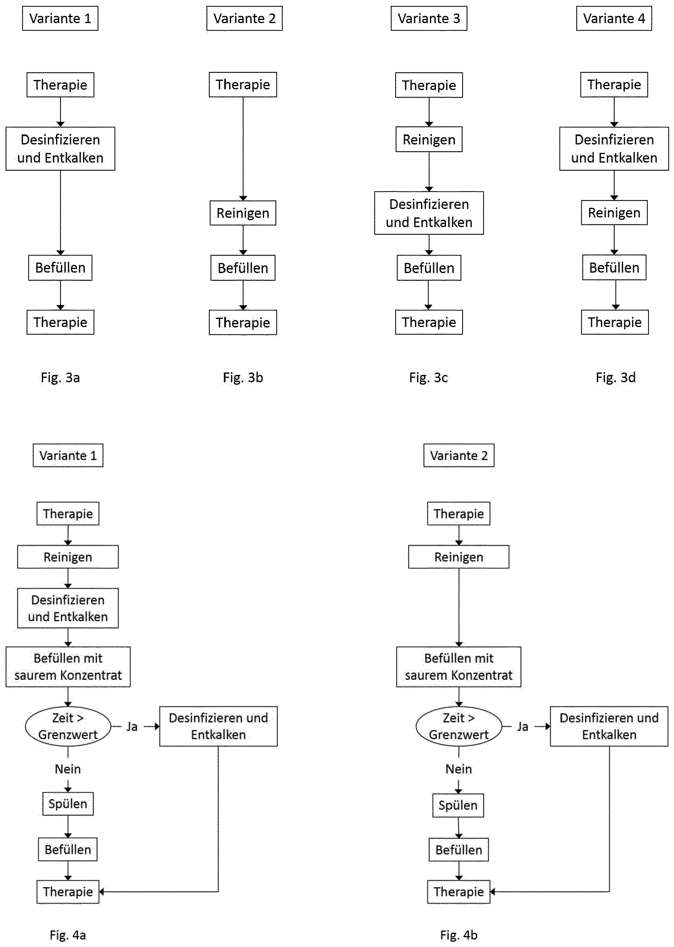

Fig. 3a zeigt eine schematische Darstellung einer ersten beispielhaften Ausführungsform eines Verfahrens zur Nach- und Vorbereitung eines Therapieablaufs an einer Vorrichtung zur extrakorporalen Blutbehandlung, das zwischen zwei Therapieabläufen durchgeführt wird. Im Anschluss an eine Therapie wird der Verfahrensschritt Desinfizieren und Entkalken durchgeführt. Da eine Reinigung nicht nach jedem Therapieablauf erforderlich ist, kann das Hydrauliksystem im Anschluss an den Verfahrensschritt Desinfizieren und Entkalken direkt von dem Verfahrensschritt Befüllen gefolgt werden. Beim Verfahrensschritt Befüllen wird das Hydrauliksystem mit Dialysierflüssigkeit, die aus hochreinem Wasser, Bicarbonat und saurem Konzentrat angemischt wird, befüllt werden und somit in den Ausgangszustand für den nächsten Therapieablauf gesetzt werden.

Fig. 3b zeigt eine schematische Darstellung einer zweiten beispielhaften Ausführungsform eines Verfahrens zur Nach- und Vorbereitung eines Therapieablaufs an einer Vorrichtung zur extrakorporalen Blutbehandlung, das zwischen zwei Therapieabläufen durchgeführt wird. Im Anschluss an eine Therapie wird direkt der Verfahrensschritt Reinigung durchgeführt, gefolgt von dem Verfahrensschritt Befüllen. Beim Verfahrensschritt Befüllen wird das Hydrauliksystem mit Dialysierflüssigkeit befüllt und somit in den Ausgangszustand für den nächsten Therapieablauf gesetzt. Der Verfahrensschritt Desinfizieren und Entkalken kann zwischen zwei Therapieabläufen übersprungen werden.

Fig. 3c zeigt eine schematische Darstellung einer dritten beispielhaften Ausführungsform eines Verfahrens zur Nach- und Vorbereitung eines Therapieablaufs an einer Vorrichtung zur extrakorporalen Blutbehandlung, das zwischen zwei Therapieabläufen durchgeführt wird. Im Anschluss an eine Therapie wird der Verfahrensschritt Reinigen durchgeführt, um das Hydrauliksystem von Schmutz und Fett zu befreien. Anschließend wird der Verfahrensschritt Desinfizieren und Entkalken durchgeführt, der vom Verfahrensschritt Befüllen gefolgt wird. Beim Verfahrensschritt Befüllen wird das Hydrauliksystem mit Dialysierflüssigkeit befüllt und somit in den Ausgangszustand für den nächsten Therapieablauf gesetzt. Dieses Verfahrens ist vorteilhaft, wenn zwischen zwei Therapieabläufen eine längere Ruhezeit geplant ist. Das Verfahren kann dann im Verfahrensschritt Desinfizieren und Entkalken vor dem Teilverfahrensschritt Ausspülen unterbrochen werden und das saure Konzentrat verbleibt bis zur Fortsetzung des Verfahrens im Hydrauliksystem. Durch das saure Klima im Hydrauliksystem wird einer Keimbildung während der Ruhezeit entgegengewirkt. Eine Schädigung der Leitungen ist durch die hohe Materialverträglichkeit des sauren Konzentrats nicht zu erwarten. Vor Beginn der nächsten Therapiesitzung kann das Verfahren fortgesetzt werden, sodass das saure Konzentrat ausgespült wird und das Hydrauliksystem mit Dialysierflüssigkeit befüllt wird.

Fig. 3d zeigt eine schematische Darstellung einer vierten beispielhaften Ausführungsform eines Verfahrens zur Nach- und Vorbereitung eines Therapieablaufs an einer Vorrichtung zur extrakorporalen Blutbehandlung, das zwischen zwei Therapieabläufen durchgeführt wird. Im Anschluss an eine Therapie wird der Verfahrensschritt Desinfizieren und Entkalken durchgeführt. Da das Hydrauliksystem in regelmäßigen Abständen von anfallenden Verschmutzungen und Fetten aus der Blutbehandlung gereinigt werden muss, wird im Anschluss an den Verfahrensschritt Desinfizieren und Entkalken der Verfahrensschritt Reinigen ausgeführt. Da Bicarbonat die Hydraulikkomponenten und den Dialysierflüssigkeitsfilter nicht angreift, kann bei Bedarf nach jeder Therapie bzw. täglich ohne Materialabnutzung gereinigt werden. Gefolgt wird der Verfahrensschritt Reinigen anschließend von dem Verfahrensschritt Befüllen, in dem das Hydrauliksystem mit Dialysierflüssigkeit befüllt und somit in den Ausgangszustand für den nächsten Therapieablauf gesetzt wird.

Fig. 4a zeigt eine schematische Darstellung einer ersten beispielhaften Ausführungsform eines Verfahrens zur Nach- und Vorbereitung eines Therapieablaufs an einer Vorrichtung zur extrakorporalen Blutbehandlung, das zwischen zwei Therapieabläufen durchgeführt wird, für einen Fall, in dem zwischen zwei Therapieabläufen eine längere Ruhezeit geplant ist. Dies kann beispielsweise die betriebsfreie Standzeit über Nacht oder über ein Wochenende sein. Nach dem ersten Therapieablauf wird der Verfahrensschritt Reinigen durchgeführt, gefolgt vom Verfahrensschritt Desinfizieren und Entkalken. Nachdem die Lösung, in der vorhandener Kalk in Form von Calciumacetat gelöst wurde, ausgespült wurde, wird der Verfahrensschritt Befüllen mit saurem Konzentrat durchgeführt. Das saure Konzentrat verbleibt im Anschluss solange im Hydrauliksystem, bis der nächste Therapieablauf stattfinden soll. Dabei wird zunächst ermittelt, ob ein definierter zeitlicher Grenzwert zwischen Ablauf des letzten und Beginn des nächsten Therapieablaufs überschritten wurde oder nicht. Wurde der Grenzwert nicht überschritten, wird das saure Konzentrat mit hochreinem Wasser aus dem Hydrauliksystem gespült und dieses anschließend mit Dialysierflüssigkeit befüllt, um die Ausgangsbedingung für den folgenden Therapieablauf zu schaffen. Wurde der Grenzwert überschritten, wird zunächst der Verfahrensschritt Desinfizieren und Entkalken wiederholt, um eine mögliche Keimbelastung zu entfernen. Alternativ kann der Teilverfahrensschritt Ausspülen im Verfahrensschritt Desinfizieren und Entkalken übersprungen werden, sodass der Verfahrensschritt Befüllen mit saurem Konzentrat nicht durchgeführt werden muss.

Fig. 4b zeigt eine schematische Darstellung einer zweiten beispielhaften Ausführungsform eines Verfahrens zur Nach- und Vorbereitung eines Therapieablaufs an einer Vorrichtung zur extrakorporalen Blutbehandlung, das zwischen zwei Therapieabläufen durchgeführt wird, für einen Fall, in dem zwischen zwei Therapieabläufen eine längere Ruhezeit geplant ist. Nach dem ersten Therapieablauf wird der Verfahrensschritt Reinigen durchgeführt, der direkt gefolgt wird vom Verfahrensschritt Befüllen mit saurem Konzentrat. Das saure Konzentrat verbleibt im Anschluss solange im Hydrauliksystem, bis der nächste Therapieablauf stattfinden soll. Dabei wird zunächst ermittelt, ob ein definierter zeitlicher Grenzwert zwischen Ablauf des letzten und Beginn des nächsten Therapieablaufs überschritten wurde oder nicht. Wurde der Grenzwert nicht überschritten, wird das saure Konzentrat mit hochreinem Wasser aus dem Hydrauliksystem gespült und dieses anschließend mit Dialysierflüssigkeit befüllt, um die Ausgangsbedingung für den folgenden Therapieablauf zu schaffen. Wurde der Grenzwert überschritten, wird zunächst der Verfahrensschritt Desinfizieren und Entkalken wiederholt, um eine mögliche Keimbelastung zu entfernen. Dieses Verfahren eignet sich insbesondere bei Therapieabläufen, zwischen denen kein Patientenwechsel stattfindet, beispielsweise Patienten mit einer Heimdialyse. Es ist auch geeignet, wenn die planmäßige Durchführung der nächsten Therapiesitzung hinter dem definierten zeitlichen Grenzwert liegt, sodass der Verfahrensschritt Desinfizieren und Entkalken nicht doppelt stattfindet.

Um die Keimbelastung des Hydrauliksystems einer Vorrichtung zur extrakorporalen Blutwäsche niedrig zu halten, ist in einer weiteren Ausführungsform eines Verfahrens zur Nach- und Vorbereitung eines Therapieablaufs an einer Vorrichtung zur extrakorporalen Blutbehandlung, das zwischen zwei Therapieabläufen durchgeführt wird, vorgesehen, dass nach Abschluss der letzten Therapie des Tages der Verfahrensschritt Befüllen mit saurem Konzentrat durchgeführt wird, auch wenn vorher keine Reinigung stattgefunden hat.

## Patentansprüche

1. Verfahren zur Nach- und Vorbereitung eines Therapieablaufs an einer Vorrichtung zur extrakorporalen Blutbehandlung; mit den Verfahrensschritten
• Desinfizieren sowie Entkalken und
• Reinigen
eines Hydrauliksystems der Vorrichtung zur extrakorporalen Blutbehandlung wobei
die Verfahrensschritte Desinfizieren und Entkalken des Hydrauliksystems in einem Kombinationsverfahrensschritt gleichzeitig durch Einsatz eines sauren Konzentrats erfolgen und der Verfahrensschritt Reinigen des Hydrauliksystems durch Einsatz einer Bicarbonatlösung erfolgt, **dadurch gekennzeichnet, dass** der Verfahrensschritt Reinigen des Hydrauliksystems weiter die Teilverfahrensschritte
• Ansaugen der Bicarbonatlösung,
• Erhitzen und Zirkulieren der Bicarbonatlösung auf mindestens 50°C,
• Zirkulieren der Bicarbonatlösung und
• Ausspülen der Bicarbonatlösung
aufweist.

2. Verfahren nach Anspruch 1, **dadurch gekennzeichnet, dass** der Kombinationsverfahrensschritt Desinfizieren und Entkalken die Teilverfahrensschritte
• Ansaugen des sauren Konzentrats,
• Erhitzen und Zirkulieren des sauren Konzentrats,
• Zirkulieren des sauren Konzentrats und
• Ausspülen des sauren Konzentrats,
aufweist.

3. Verfahren nach Anspruch 2, **dadurch gekennzeichnet, dass** abhängig von einer vorhergehenden Therapiezeit und einer für diese Therapie eingestellten Dialysierflüssigkeitszusammensetzung eine zum Lösen von Verkalkungen und zum Desinfizieren des Hydrauliksystems tatsächlich benötigte Menge sauren Konzentrats von einem Anwender in eine Gerätesteuerung
eingegeben oder automatisch von dieser berechnet wird und in dem Teilverfahrensschritt Ansaugen des sauren Konzentrats die tatsächlich benötigte Menge sauren Konzentrats angesaugt wird.

4. Verfahren nach Anspruch 2 oder 3, **dadurch gekennzeichnet, dass** in dem Teilverfahrensschritt Erhitzen und Zirkulieren des sauren Konzentrats das saure Konzentrats auf mindestens 80°C erhitzt wird.

5. Verfahren nach einem der Ansprüche 2 bis 4, **dadurch gekennzeichnet, dass** abhängig von einer vorangegangenen Therapiedauer und einer für diese Therapie eingesetzten Dialysierflüssigkeitszusammensetzung eine zum vollständigen Entkalken und Desinfizieren tatsächlich benötigte Zirkulationszeit von einem Anwender in eine Gerätesteuerung eingegeben oder automatisch von dieser berechnet wird und der Teilverfahrensschritt Zirkulieren des sauren Konzentrats für die tatsächlich benötigte Zirkulationszeit durchgeführt wird.

6. Verfahren nach Anspruch 2 bis 5, **dadurch gekennzeichnet, dass** der Teilverfahrensschritt Ausspülen des sauren Konzentrats bis zum Erreichen eines definierten Grenzwerts durchgeführt wird.

7. Verfahren nach Anspruch 1, **dadurch gekennzeichnet, dass** abhängig von einer Therapiezeit seit der letzten Reinigung eine zum Lösen von Verschmutzungen tatsächlich benötigte Menge Bicarbonatlösung von einem Anwender in eine Gerätesteuerung eingegeben oder automatisch von dieser berechnet wird und in dem Teilverfahrensschritt Ansaugen der Bicarbonatlösung die tatsächlich benötigte Menge Bicarbonatlösung angesaugt wird.

8. Verfahren nach Anspruch 1 oder 7, **dadurch gekennzeichnet, dass** in dem Teilverfahrensschritt Erhitzen und Zirkulieren der Bicarbonatlösung die Bicarbonatlösung auf 85°C erhitzt wird.

9. Verfahren nach Anspruch 1 oder 7, **dadurch gekennzeichnet, dass** in dem Teilverfahrensschritt Erhitzen und Zirkulieren der Bicarbonatlösung die Bicarbonatlösung auf den Siedepunkt erhitzt wird.

10. Verfahren nach einem der Ansprüche 1, 8 oder 9, **dadurch gekennzeichnet, dass** abhängig von einer vorangegangenen Therapiezeit, in der keine Reinigung stattgefunden hat, eine zum vollständigen Reinigen und Entfetten tatsächlich benötigte Zirkulationszeit von einem Anwender in eine Gerätesteuerung eingegeben oder automatisch von dieser berechnet wird und der Teilverfahrensschritt Zirkulieren der Bicarbonatlösung für die tatsächlich benötigte Zirkulationszeit durchgeführt wird.

11. Verfahren nach einem der Ansprüche 1 oder 8 bis 10, **dadurch gekennzeichnet, dass**
der Teilverfahrensschritt Ausspülen der Bicarbonatlösung bis zum Erreichen eines definierten Grenzwerts durchgeführt wird.

12. Verfahren nach einem der vorangehenden Ansprüche, **gekennzeichnet durch** die weiteren Verfahrensschritte
• Spülen und
• Befüllen, wobei
der Verfahrensschritt Befüllen als letzter Verfahrensschritt vor Beginn eines nächsten Therapieablaufs mit einer Dialysierflüssigkeit durchgeführt wird und wobei in bevorzugter Weise in einem Fall, in dem der nächste Therapieablauf nicht direkt im Anschluss an das Verfahren folgt, das Hydrauliksystem mit sauren Konzentrat befüllt wird und das saure Konzentrat in dem Hydrauliksystem verbleibt und
das Hydrauliksystem direkt vor Beginn des nächsten Therapieablaufs gespült und mit Dialysierflüssigkeit befüllt wird.

13. Verfahren nach Anspruch 12, **dadurch gekennzeichnet, dass** nach Überschreiten einer definierten Zeitspanne, in der kein weiterer Therapieablauf stattgefunden hat, vor Beginn des nächsten Therapieablaufs der Verfahrensschritt Desinfizieren und Entkalken erneut durchgeführt wird.

14. Verfahren nach einem der vorangehenden Ansprüche, **dadurch gekennzeichnet, dass**
das saure Konzentrat einen Anteil Acetat und/oder Citrat enthält.

15. Verfahren nach Anspruch 2 oder 3, **dadurch gekennzeichnet, dass** in dem Teilverfahrensschritt Erhitzen und Zirkulieren des sauren Konzentrats das saure Konzentrats auf über 85°C erhitzt wird.

16. Verfahren nach Anspruch 2 oder 3, **dadurch gekennzeichnet, dass** in dem Teilverfahrensschritt Erhitzen und Zirkulieren des sauren Konzentrats das saure Konzentrats auf ca.den Siedepunkt erhitzt wird.

## Claims

1. Method for post-processing and preparing a treatment cycle on a device for extracorporeal blood treatment with the process steps of
• disinfecting, descaling and
• cleaning
a hydraulic system of the device for extracorporeal blood treatment, wherein
the process steps of disinfecting and descaling the hydraulic system is carried out at the same time in a combination process step by using an acidic concentrate and the process step of cleaning the hydraulic system being carried out by using a bicarbonate solution, **characterised in that** the process step of cleaning the hydraulic system further includes the partial process steps of
• suctioning the bicarbonate solution,
• heating up and circulating the bicarbonate solution to at least 50°C
• circulating the bicarbonate solution and
• flushing out the bicarbonate solution.

2. Method according to claim 1, **characterised in that** the combination process step of disinfecting and descaling includes the partial process steps of
• suctioning the acidic concentrate,
• heating up and circulating the acidic concentrate,
• circulating the acidic concentrate and
• flushing out the acidic concentrate.

3. Method according to claim 2, **characterised in that,** depending on a preceding treatment time and a dialysis fluid composition used for this treatment, an amount of acidic concentrate actually required to dissolve lime scale and disinfect the hydraulic system is input by a user into an equipment control or automatically calculated by it and the actual amount of acidic concentrate required is suctioned in the partial process step of suctioning the acidic concentrate.

4. Method according to claim 2 or 3, **characterised in that,** in the partial process step of heating up and circulating the acidic concentrate, the acidic concentrate is heated up to at least 80°C.

5. Method according to any of claims 2 to 4, **characterised in that,** depending on a preceding treatment duration and a dialysis fluid composition used for this treatment, a circulation time actually required for the complete descaling and disinfecting is input by a user into an equipment control or is automatically calculated by it and the partial process step of circulating the acidic concentrate is carried out for the actual circulation time required.

6. Method according to claims 2 to 5, **characterised in that** the partial process step of flushing out the acidic concentrate is carried out until a specified limit value is reached.

7. Method according to claim 1, **characterised in that,** depending on a treatment time since the last clean, an amount of bicarbonate solution actually required to dissolve dirt is input by a user into an equipment control or is automatically calculated by it and the actual amount of bicarbonate solution required is suctioned in the partial process step of suctioning the bicarbonate solution.

8. Method according to claim 1 or 7, **characterised in that,** in the partial process step of heating up and circulating the bicarbonate solution, the bicarbonate solution is heated up to 85°C.

9. Method according to claim 1 or 7, **characterised in that,** in the partial process step of heating up and circulating the bicarbonate solution, the bicarbonate solution is heated up to boiling point.

10. Method according to any of claims 1, 8 or 9, **characterised in that,** depending on a preceding treatment time in which no cleaning took place, a circulation time actually required for the complete cleaning and degreasing is input by a user into an equipment control or is automatically calculated by it and the partial process step of circulating the bicarbonate solution is carried out for the actual circulation time required.

11. Method according to any of claims 1 or 8 to 10, **characterised in that**
the partial process step of flushing out the bicarbonate solution is carried out until a specified limit value is reached.

12. Method according to any of the preceding claims, **characterised by** the further process steps of
• rinsing and
• filling, wherein
the process step of filling is carried out as last process step before the start of a next treatment cycle with a dialysis fluid and wherein it is preferable, in a case in which the next treatment cycle does not immediately follow the procedure, for the hydraulic system to be filled with acidic concentrate and the acidic concentrate to remain in the hydraulic system and
the hydraulic system to be rinsed and filled with dialysis fluid directly before the next treatment cycle.

13. Method according to claim 12, **characterised in that,** when a defined time frame in which no further treatment cycle has taken place is exceeded, the process step of disinfecting and descaling is carried out again before the start of the next treatment cycle.

14. Method according to one of the preceding claims, **characterised in that** the acidic concentrate contains a proportion of acetate and/or citrate.

15. Method according to claim 2 or 3, **characterised in that,** in the partial process step of heating up and circulating the acidic concentrate, the concentrate is heated up to over 85°C.

16. Method according to claim 2 or 3, **characterised in that,** in the partial process step of heating up and circulating the acidic concentrate, the acidic concentrate is heated up to around boiling point.

## Revendications

1. Procédé de post-traitement et de préparation d'une procédure thérapeutique au niveau d'un dispositif de traitement extracorporel du sang, avec les étapes consistant à
• désinfecter et détartrer et
• nettoyer
un système hydraulique du dispositif de traitement extracorporel du sang, dans lequel
les étapes de désinfection et de détartrage du système hydraulique sont mises en oeuvre simultanément lors d'une étape combinée en utilisant un concentré acide, et l'étape de nettoyage du système hydraulique est mise en oeuvre en utilisant une solution de bicarbonate, **caractérisé en ce que** l'étape de nettoyage du système hydraulique inclut en outre les étapes partielles consistant à
• aspirer la solution de bicarbonate,
• chauffer et mettre en circulation la solution de bicarbonate à au moins 50°C,
• mettre en circulation la solution de bicarbonate et
• rincer la solution de bicarbonate.

2. Procédé selon la revendication 1, **caractérisé en ce que** l'étape combinée de désinfection et de détartrage inclut les étapes partielles consistant à
• aspirer le concentré acide,
• chauffer et mettre en circulation le concentré acide,
• mettre en circulation le concentré d'acide et
• rincer le concentré acide.

3. Procédé selon la revendication 2, **caractérisé en ce que**, en fonction d'un temps de procédure thérapeutique précédent et d'une composition de liquide de dialyse ajustée pour ladite procédure thérapeutique, une quantité de concentré acide réellement nécessaire pour dissoudre les calcifications et désinfecter le système hydraulique est entrée par un utilisateur dans une commande d'équipement ou calculée automatiquement par celle-ci, et la quantité réellement nécessaire de concentré acide est aspirée lors de l'étape partielle d'aspiration du concentré acide.

4. Procédé selon la revendication 2 ou 3, **caractérisé en ce que** le concentré acide est chauffé à au moins 80°C lors de l'étape partielle de chauffage et de mise en circulation du concentré acide.

5. Procédé selon l'une quelconque des revendications 2 à 4, **caractérisé en ce que,** en fonction d'une durée de procédure thérapeutique précédente et d'une composition de liquide de dialyse utilisée pour ladite procédure thérapeutique, un temps de circulation réellement nécessaire pour un détartrage complet et une désinfection complète est entré par un utilisateur dans une commande d'équipement ou calculé automatiquement par celle-ci et l'étape partielle de mise en circulation du concentré acide est mise en oeuvre pendant le temps de circulation réellement nécessaire.

6. Procédé selon la revendication 2 à 5, **caractérisé en ce que** l'étape partielle de rinçage du concentré acide est mise en oeuvre jusqu'à atteindre une valeur limite définie.

7. Procédé selon la revendication 1, **caractérisé en ce que**, en fonction d'un temps de procédure thérapeutique écoulé depuis le dernier nettoyage, une quantité de solution de bicarbonate réellement nécessaire pour dissoudre des salissures est entrée par un utilisateur dans une commande d'équipement ou automatiquement calculée par celle-ci et la quantité de solution de bicarbonate réellement nécessaire est aspirée lors de l'étape partielle d'aspiration de la solution de bicarbonate.

8. Procédé selon la revendication 1 ou 7, **caractérisé en ce que** la solution de bicarbonate est chauffée à 85°C lors de l'étape partielle de chauffage et de mise en circulation de la solution de bicarbonate.

9. Procédé selon la revendication 1 ou 7, **caractérisé en ce que** la solution de bicarbonate est chauffée jusqu'au point d'ébullition lors de l'étape partielle de chauffage et de mise en circulation de la solution de bicarbonate.

10. Procédé selon l'une quelconque des revendications 1, 8 ou 9, **caractérisé en ce que**, en fonction d'un temps de procédure thérapeutique précédent pendant lequel aucun nettoyage n'a été effectué, un temps de circulation réellement nécessaire pour un nettoyage complet et un dégraissage complet est entré par un utilisateur dans une commande d'équipement ou calculé automatiquement par celle-ci et l'étape partielle de mise en circulation de la solution de bicarbonate est mise en oeuvre pendant le temps de circulation réellement nécessaire.

11. Procédé selon l'une quelconque des revendications 1 ou 8 à 10, **caractérisé en ce que**
l'étape partielle de rinçage de la solution de bicarbonate est mise en oeuvre jusqu'à atteindre une valeur limite définie.

12. Procédé selon l'une quelconque des revendications précédentes, **caractérisé par** les autres étapes consistant à
• rincer et
• remplir, dans lequel
l'étape de remplissage est mise en oeuvre avec un liquide de dialyse en tant que dernière étape avant le début d'une procédure thérapeutique suivante, et dans lequel de manière préférée, dans le cas où la procédure thérapeutique suivante n'est pas immédiatement consécutive au sein du procédé, le système hydraulique est rempli avec du concentré acide et le concentré acide reste dans le système hydraulique et
le système hydraulique est rincé et est rempli de liquide de dialyse immédiatement avant le début de la procédure thérapeutique suivante.

13. Procédé selon la revendication 12, **caractérisé en ce que** l'étape de désinfection et de détartrage est à nouveau mise en oeuvre avant le début de la procédure thérapeutique suivante après un dépassement d'une plage temporelle définie pendant laquelle aucune autre procédure thérapeutique n'a eu lieu.

14. Procédé selon l'une quelconque des revendications précédentes, **caractérisé en ce que**
le concentré acide contient une proportion d'acétate et/ou de citrate.

15. Procédé selon la revendication 2 ou 3, **caractérisé en ce que** le concentré acide est chauffé à plus de 85°C lors de l'étape partielle de chauffage et de mise en circulation.

16. Procédé selon la revendication 2 ou 3, **caractérisé en ce que** le concentré acide est chauffé jusqu'à environ le point d'ébullition lors de l'étape partielle de chauffage et de mise en circulation du concentré acide.
